# FASCICULE DE BREVET EUROPEEN

(11) **EP 3 823 446 B1**
(45) Date de publication et mention de la délivrance du brevet: **20.09.2023**
(21) Numéro de dépôt: 19739996.7
(22) Date de dépôt: 17.07.2019
(51) Int. Cl.: A01N 1/02, A61F 2/00, A61F 2/14

(54) **DISPOSITIF DE MAINTIEN D'UN TISSU CORNEEN POUR SON TRAITEMENT PHOTONIQUE**
VORRICHTUNG ZUR AUFNAHME VON HORNHAUTGEWEBE ZUR PHOTONENBEHANDLUNG DAVON
DEVICE FOR HOLDING CORNEAL TISSUE FOR PHOTON TREATMENT THEREOF

(30) Priorité: 17.07.2018 FR 1870835
(43) Date de publication de la demande: 26.05.2021
(73) Titulaire: Université Jean Monnet Saint-Étienne, 42100 Saint Étienne (FR); Centre national de la recherche scientifique, 75016 Paris (FR); Centre Hospitalier Universitaire, 42100 Saint Etienne (FR); Manutech-USD, 42000 Saint Etienne (FR)
(72) Inventeur: GAIN, Philippe, 69009 LYON (FR); THURET, Gilles, 42330 SAINT BONNET LES OULES (FR); MAUCLAIR, Cyril, 42100 SAINT ETIENNE (FR); JUMELLE, Clotilde, 62580 FRESNOY EN GOHELLE (FR); EGAUD, Gregory, 42660 JONZIEUX (FR)
(74) Mandataire: Be IP
(86) Numéro de dépôt international: PCT/EP2019/069192
(87) Numéro de publication internationale: WO 2020/016270

(56) Documents cités:
- WO-A1-2014/140434
- US-A1- 2008 294 149

## Description

### DOMAINE DE L'INVENTION

La présente invention concerne le domaine technique général des dispositifs médicaux pour le traitement et/ou la découpe d'un tissu cornéen ex-vivo humain ou animal.

Un tel dispositif peut être utilisé pour réaliser des expérimentations sur la cornée et/ou pour découper/traiter une cornée en vue d'une greffe.

### ARRIERE PLAN DE L'INVENTION

La cornée est un élément indispensable à la vision d'un patient : elle constitue en effet la fenêtre par laquelle les images du monde extérieur pénètrent dans l'oeil.

La cornée d'un patient peut subir différents dommages - engendrant une perte totale ou partielle de vision - liés à différentes affections dont le patient est atteint, telles qu'une dystrophie de cornée, un kératocône, ou une opacité ou une perforation faisant suite à une infection de la cornée.

Lorsque la cornée est devenue opaque, déformée ou qu'elle est perforée, le patient est susceptible de bénéficier d'une greffe. Cette greffe peut être totale ou partielle, et de différentes formes.

Par ailleurs, il est désormais possible de modifier la puissance dioptrique de la cornée en y insérant une greffe sous forme de lenticule de forme choisie. Il est aussi possible d'insérer une lamelle cornéenne très fine recouverte de cellules endothéliales en vue d'une greffe endothéliale (bioingénierie cornéenne).

La greffe partielle de cornée consiste à greffer un fragment de cornée sain issu d'un donneur - ou implant cornéen - en remplacement d'une portion de cornée malade du receveur.

Afin de réaliser l'implant cornéen à greffer, il a déjà été proposé différents dispositifs de découpe.

Le document US 2017/319329 décrit notamment un système pour former des implants cornéens incluant un premier appareil de coupe et un deuxième appareil de coupe.

Le premier appareil de coupe est configuré pour couper une cornée d'un donneur et former une portion de cornée. Plus précisément le premier appareil de coupe est configuré pour couper la cornée du donneur le long d'un axe s'étendant entre une surface antérieure et une surface postérieure de la cornée.

Le deuxième appareil de coupe est configuré pour former une pluralité de lenticules à partir de la portion de cornée en formant une série de coupes transversales dans la portion de cornée. Le tissu cornéen entre deux plans de coupe consécutifs constitue un lenticule. Ce lenticule est ensuite utilisé pour former l'implant cornéen.

Le deuxième appareil de coupe consiste en un boitier incluant :
- un fond,
- des parois latérales,
- une fenêtre transparente à un rayonnement lumineux opposée au fond.

Le boitier comprend également une chambre destinée à recevoir la portion de cornée à découper en lenticules. Un flux d'air traverse le boitier entre la fenêtre transparente et la chambre, et un fluide hydratant circule dans le boitier entre son fond et la chambre afin de maintenir la face postérieure de la cornée immergée dans le fluide.

La portion de cornée n'étant pas contrainte mécaniquement entre ses faces antérieure et postérieure, celle-ci est relâchée. Pour permettre la réalisation des plans de coupe, la fenêtre transparente est mobile en translation selon une direction perpendiculaire au fond afin de permettre d'aplanir la portion de cornée par l'avant préalablement à la réalisation des plans de coupe à l'aide d'un rayonnement L.A.S.E.R.

Un inconvénient de ce type de dispositif est qu'il ne permet pas de réaliser des plans de coupe de précision. En effet, la portion de cornée peut subir des déplacements antéro-postérieurs, en particulier lors de l'interaction L.A.S.E.R./cornée, préjudiciable à la précision de focalisation du faisceau L.A.S.E.R.

Un autre inconvénient de ce type de dispositif est que la qualité des plans de coupe diminue avec la profondeur. En effet, au plus le plan focal est profond dans la cornée, au plus l'efficacité du faisceau L.A.S.E.R. diminue. Par ailleurs, le nombre de lenticules réalisable avec ce type de dispositif est limité.

Un autre inconvénient encore de ce type de dispositif est que la chambre de réception de la portion de cornée n'est pas en vase clos (cornée dans un environnement non étanche). Le dispositif doit donc être manipulé dans un environnement contrôlé (poste de sécurité microbiologique, salle blanche, bloc opératoire).

Le document WO2014/140434 décrit un dispositif de conservation d'un prélèvement cornéen comprenant un couvercle endothélial, un couvercle épithélial, une pièce intermédiaire entre les couvercles endothélial et épithélial, et un système de verrouillage. Dans le dispositif selon WO2014/140434, la portion de cornée n'est pas contrainte mécaniquement entre ses faces antérieure et postérieure. Ce dispositif de conservation n'est en outre pas adapté à la formation d'implants cornéens par découpe de la portion de cornée.

Le document US 2008/294149 décrit une chambre d'observation pour la préservation du tissu cornéen. La chambre comprend un récipient et un couvercle. Le récipient comprend un panier cornéen formé par une pluralité de griffes pour supporter le tissu cornéen. Les griffes sont dimensionnées de manière à pouvoir recevoir des cornées de différentes tailles. Dans le dispositif selon US 2008/294149, la portion de cornée n'est pas contrainte mécaniquement entre ses faces antérieure et postérieure. Ce dispositif de conservation n'est en outre pas adapté à la formation d'implants cornéens par découpe de la portion de cornée.

Un but de la présente invention est de proposer un dispositif de maintien d'un tissu cornéen pour son traitement et/ou sa découpe permettant de pallier au moins l'un des inconvénients précités.

### BREVE DESCRIPTION DE L'INVENTION

A cet effet, l'invention propose un dispositif de maintien d'un tissu cornéen humain ou animal préalablement prélevé pour son traitement photonique à partir d'un rayonnement électromagnétique, ***remarquable en* ce *que*** le dispositif de maintien comprend :
- un empilement d'éléments selon un axe longitudinal A-A' du dispositif, ledit empilement comportant :
   ∘ une première plaque transparente au rayonnement électromagnétique,
   ∘ un joint périphérique positionné sur la première plaque, le joint périphérique étant destiné à s'étendre autour du tissu cornéen (sans être nécessairement en contact avec le tissu cornéen),
   ∘ une deuxième plaque transparente au rayonnement électromagnétique sur le joint périphérique,
- un système de blocage de l'empilement d'éléments apte à plaquer les première et deuxième plaques contre le tissu cornéen pour appliquer une contrainte mécanique sur des faces antérieure et postérieure du tissu cornéen.

Le dispositif décrit ci-dessus permet de traiter/découper une cornée dans des conditions stériles.

En travaillant la cornée par les deux faces, le dispositif selon l'invention permet de limiter la puissance du faisceau LASER nécessaire lors de la formation de fragments à partir de la cornée. La moitié des découpes étant effectuées en passant par une face et la seconde moitié en passant par la face opposée, le faisceau LASER n'a au maximum que la moitié de la cornée à traverser. L'énergie employée est ainsi plus faible, les tissus ne s'altèrent pas ce qui améliore la qualité du fragment.

Par ailleurs, la contrainte mécanique appliquée par les première et deuxième plaques sur les faces antérieure et postérieure du tissu cornéen permet de limiter les risques de déplacement antéro-postérieur du tissu cornéen lors de l'application du faisceau électromagnétique, en particulier lors de l'interaction entre le faisceau photonique et le tissu cornéen. Cette contrainte mécanique permet aussi la réalisation de plan de découpe plus fin et plus précis en forçant les bulles créer par le faisceau à s'évacuer des tissus ceci permet de diminuer l'épaisseur des fragments et par conséquent d'augmenter la quantité d'implants réalisables dans une même cornée.

Ces fragments découpés sont destinés à être utilisés pour diverses applications : reconstruction in vitro de greffons endothéliaux ; patch pour chirurgie réparatrice ; implant intra cornéen pour modifier les propriétés réfractives de la cornée, etc. Un exemple d'utilisation de l'invention est la découpe de multiples lamelles (jusqu'à 20) dans une seule cornée par L.A.S.E.R. femtoseconde. Un tel nombre de lamelles n'a jamais été obtenu avec les dispositifs de l'art antérieur.

Des aspects préférés mais non limitatifs du dispositif selon l'invention sont les suivants :
- les première et deuxième plaques peuvent être parallèles, la distance entre les première et deuxième plaques étant comprise entre 100 µm et 1800 µm, préférentiellement entre 400 µm et 1500 µm, et encore plus préférentiellement entre 500 µm et 700 µm,
- l'épaisseur du joint périphérique peut être comprise entre 100 µm et 1800 µm, préférentiellement entre 400 µm et 1500 µm, et encore plus préférentiellement entre 500 µm et 700 µm, ladite épaisseur du joint définissant la distance entre les première et deuxième plaques représentative de la contrainte mécanique appliquée sur le tissu cornéen par lesdites plaques,
- le système de blocage de l'empilement d'éléments peut être apte à plaquer les première et deuxième plaques contre le joint périphérique de sorte à espacer les première et deuxième plaques d'une distance comprise entre 100 µm et 1800 µm, préférentiellement entre 400 µm et 1500 µm, et encore plus préférentiellement entre 500 µm et 700 µm,
- le système de blocage peut comporter un cadre destiné à entourer au moins partiellement les bords des première et deuxième plaques, le cadre étant apte à :
   ∘ induire sur la première plaque l'application d'une force parallèle à l'axe longitudinal et tendant à déplacer la première plaque vers la deuxième plaque,
   ∘ induire sur la deuxième plaque l'application d'une force parallèle à l'axe longitudinal et tendant à déplacer la deuxième plaque vers la première plaque,
- le cadre peut être composé d'au moins une première et deuxième portions, chaque portion incluant un plateau supérieur, un plateau inférieur et une paroi latérale entre les plateaux supérieur et inférieur :
   ∘ la face interne du plateau supérieur étant destinée à venir en regard de la face externe de la première plaque,
   ∘ la face interne du plateau inférieur étant destinée à venir en regard de la face externe de la deuxième plaque, et
   ∘ la face interne de la paroi latérale étant destinée à venir en regard des bords des plaques et de la face externe du joint,
- le système de blocage peut comporter en outre au moins un élément de serrage, tel qu'une vis, chaque élément de serrage étant destiné à coopérer avec un trou respectif ménagé dans le plateau supérieur et/ou dans le plateau inférieur d'une portion du cadre,
- chaque élément de serrage peut être déplaçable pour régler la distance entre les première et deuxième plaques, ladite distance étant comprise entre 100 µm et 1800 µm, préférentiellement entre 400 µm et 1500 µm, et encore plus préférentiellement entre 500 µm et 700 µm,
- les première et deuxième portions de cadre incluent des moyens de fixation pour permettre la solidarisation desdites portions ensemble,
- le dispositif peut comprendre en outre une entretoise positionnée entre les première et deuxième plaques, le joint périphérique s'étendant autour de l'entretoise,
- l'épaisseur de l'entretoise peut être comprise entre 100 µm et 1800 µm, préférentiellement entre 400 µm et 1500 µm, et encore plus préférentiellement entre 500 µm et 700 µm, ladite épaisseur de l'entretoise définissant la distance entre les première et deuxième plaques représentative de la contrainte mécanique appliquée sur le tissu cornéen par lesdites plaques, ladite distance étant comprise entre 100 µm et 1800 µm, préférentiellement entre 400 µm et 1500 µm, et encore plus préférentiellement entre 500 µm et 700 µm,
- l'entretoise peut comporter au moins un orifice principal définissant, avec les faces internes des première et deuxième plaques, un logement destiné à contenir le tissu cornéen,
- l'entretoise peut comporter au moins un compartiment de dégazage,
- l'entretoise peut comporter au moins un canal de connexion s'étendant entre l'orifice principal et le compartiment de dégazage pour l'acheminement de bulles de gaz formées dans le tissu cornéen.

### BREVE DESCRIPTION DES DESSINS

D'autres avantages et caractéristiques du dispositif selon l'invention ressortiront mieux de la description qui va suivre de plusieurs variantes d'exécution, données à titre d'exemples non limitatifs, à partir des dessins annexés sur lesquels :
- La figure 1 est une vue en perspective éclatée du dispositif de maintien d'un tissu cornéen,
- La figure 2 est une vue en coupe du dispositif de maintien une fois assemblé,
- La figure 3 est une vue en coupe d'une variante de réalisation du dispositif de maintien une fois assemblé,
- Les figures 4A(monoplace) et 4B(biplace) sont des vues de dessus d'exemples d'entretoise du dispositif de maintien,
- La figure 5 est une vue de dessus d'un joint périphérique du dispositif de maintien,
- La figure 6 est une vue de dessus d'un système de blocage du dispositif de maintien,
- La figure 7 est une vue en coupe d'une portion du système de blocage illustré à la figure 6.

### DESCRIPTION DETAILLEE DE L'INVENTION

On va maintenant décrire différents exemples de dispositif de maintien d'un tissu cornéen en référence aux figures. Dans ces différentes figures, les éléments équivalents sont désignés par la même référence numérique.

Dans la suite de la description, le dispositif de maintien selon l'invention sera présenté en référence à la découpe d'un tissu cornéen (i.e. une cornée ou une partie de cornée comme un disque central préalablement trépané), étant bien entendu que l'utilisation du dispositif ne se limite pas à la découpe de fragments cornéens.

Notamment, le dispositif décrit dans la suite peut être utilisé pour réaliser un traitement photonique du tissu cornéen, comme une réticulation (ou *« cross-linking* » selon la terminologie anglo-saxonne) du collagène par un agent photo-sensibilisant activé par rayonnement lumineux (par exemple riboflavine et UVA), afin de modifier ses propriétés biomécaniques (durcissement). Le lecteur appréciera que le *« cross-linking »* peut également être réalisé dans le dispositif à l'aide d'un agent chimique en dehors de tout rayonnement électromagnétique (l'intérêt du dispositif étant alors de réticuler la cornée sous contrainte puis de la découper dans le même dispositif sans avoir à la déconditionner entre temps)

### 1. Généralités

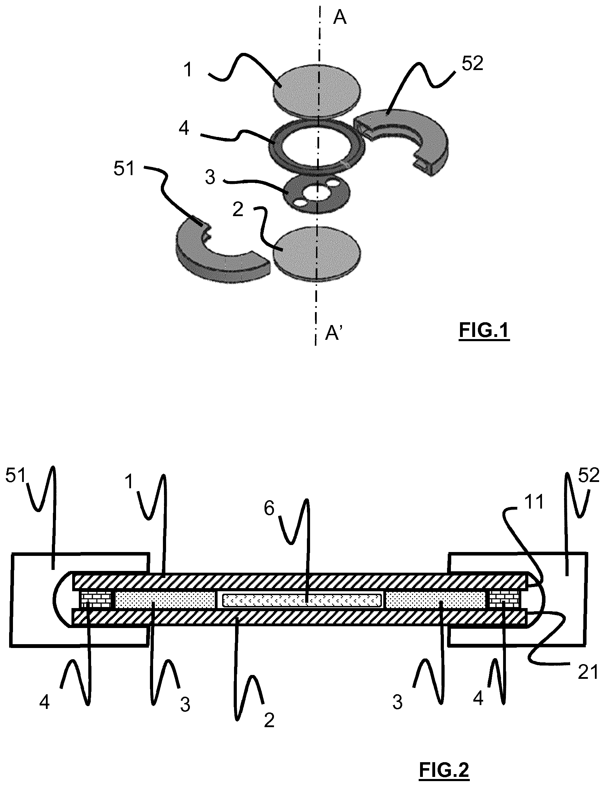

En référence aux figures 1 et 2, on a illustré un exemple de dispositif de maintien d'un tissu cornéen selon l'invention.

Le dispositif comprend :
- des première et deuxième plaques 1, 2 transparentes à un rayonnement électromagnétique,
- éventuellement une entretoise 3 destinée à être positionnée entre les première et deuxième plaques 1, 2,
- un joint périphérique 4 destiné à être positionné entre les première et deuxième plaques, le joint périphérique s'étendant autour de l'entretoise,
- un système de blocage pour l'assemblage des plaques 1, 2, de l'entretoise 3 et du joint périphérique 4.

### 1.1. Plaques transparentes

Chaque plaque 1, 2 est constituée dans un (ou plusieurs) matériau(x) biocompatible(s), stérilisable(s) et transparent(s) à un rayonnement électromagnétique émis par une source de rayonnement - telle qu'un L.A.S.E.R. (acronyme de l'anglais *« Light Amplification by Stimulated Emission of Radiation* », signifiant *« amplification de la lumière par émission stimulée de rayonnement »* en français) ou tout autre type de source de rayonnement connue de l'homme du métier pour le traitement du tissu cornéen.

Dans le mode de réalisation illustré à la figure 1, chaque plaque 1, 2 est réalisée dans un unique matériau, comme du verre ou du poly(méthacrylate de méthyle) ou tout autre matériau connu de l'homme du métier.

Dans certaines variantes de réalisation, chaque plaque 1, 2 peut être composée d'une superposition de couches de matériaux différents. Par exemple, dans une variante de réalisation, chaque plaque 1, 2 est composée d'une couche de matériau rigide (tel que du verre) s'étendant entre deux couches de matériau souple (par exemple à base de silicone) :
- la couche de matériau rigide permet d'augmenter la résistance mécanique de la plaque 1, 2, tandis que
- les couches de matériau souple permettent de limiter les risques de dispersion de morceaux de la couche de matériau rigide en cas de brisure de celle-ci.

Chaque plaque 1, 2 peut également comprendre des renforts pour augmenter sa résistance mécanique. Les renforts s'étendent par exemple au niveau des bords 11, 21 de la plaque 1, 2. Les renforts peuvent consister en des tiges en matériau rigide - tel que du titane ou de l'acier inoxydable ou tout autre métal biocompatible connu de l'homme du métier - intégrées dans la plaque 1, 2.

En variante, les renforts peuvent être dans le même matériau que la plaque 1, 2. Par exemple, les renforts peuvent consister en une (ou plusieurs) zone(s) périphérique(s) de la plaque 1, 2 ayant une (des) épaisseur(s) supérieure(s) à l'épaisseur d'une zone centrale de la plaque 1, 2. Ainsi, la plaque 1, 2 peut comprendre des zones épaisses pour renforcer sa résistance mécanique et des zones amincies pour une meilleure transmission du rayonnement électromagnétique.

Chaque plaque 1, 2 peut s'étendre sensiblement dans un plan ou être concave/convexe, la courbure (ou l'absence de courbure) de chaque plaque 1, 2 dépendant de l'application visée. Dans tous les cas, les première et deuxième plaques sont destinées à s'étendre parallèlement l'une à l'autre. On entend, dans le cadre de la présente invention, par *« plaques planes*/*concaves*/*convexes parallèles »,* des plaques dont l'espacement est constant en tout point. Ainsi, la distance entre les première et deuxième plaques est constante, et comprise entre 100 µm et 1800 µm, préférentiellement entre 400 µm et 1500 µm, et encore plus préférentiellement entre 500 µm et 700 µm. Ceci permet de contraindre mécaniquement les faces antérieure et postérieure du tissu cornéen 6 pour assurer son blocage en position dans le dispositif de maintien.

Dans le mode de réalisation illustré à la figure 1, chaque plaque 1, 2 est de forme circulaire. Toutefois, il est bien évident pour l'homme du métier que d'autres formes sont envisageables pour chaque plaque 1, 2 (carrée, rectangulaire, triangulaire, etc.).

### 1.2. Entretoise

L'entretoise 3 constitue une pièce intermédiaire destinée à être positionnée entre les première et deuxième plaques 1, 2. Elle permet de maintenir une distance prédéfinie entre les première et deuxième plaques 1, 2.

L'entretoise a plusieurs fonctions :
o elle limite le déplacement du tissu cornéen en XY (i.e. plan perpendiculaire à un axe longitudinal A-A' du dispositif)
o elle permet un repérage facile de sa position par les instruments de traitement par rayonnement électromagnétique,
∘ elle impose la distance entre les 2 plaques dans le cas où le système (simple) ne comporte pas de vis micrométrique de réglage de l'espacement entre les plaques.

L'entretoise 3 est préférentiellement rigide. Toutefois, l'entretoise 3 peut également être élastiquement déformable. L'entretoise 3 est par exemple réalisée dans un matériau biocompatible et stérilisable, notamment à base de silicone.

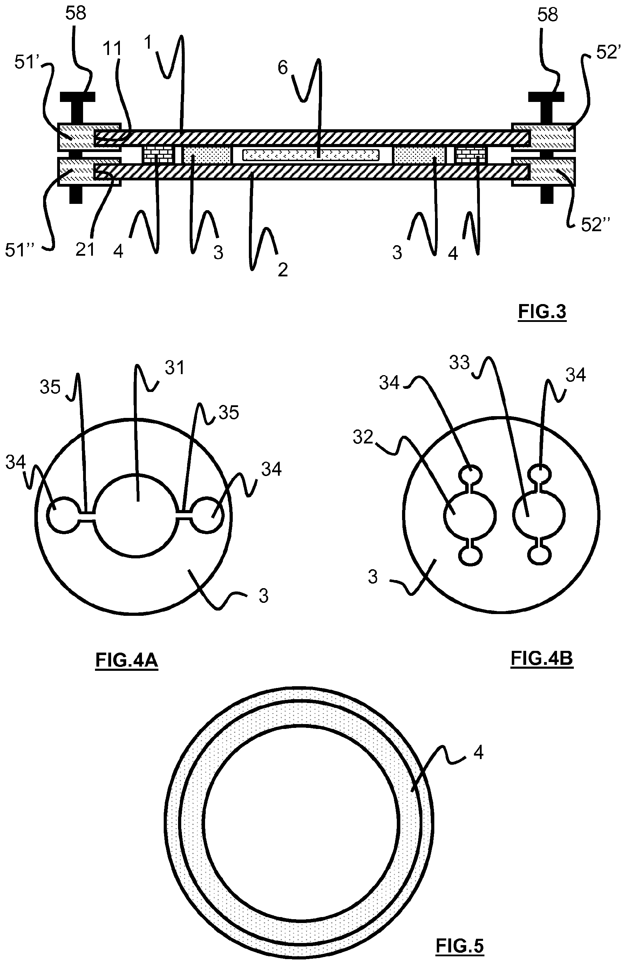

Dans le mode de réalisation illustré à la figure 4A, l'entretoise 3 comprend un orifice principal 31. L'orifice central peut être circulaire ou présenter tout autre forme souhaitée en fonction de l'application (carrée, rectangulaire, etc.). La paroi latérale de l'orifice principal 31 définit, avec les faces internes des première et deuxième plaques 1, 2, un logement destiné à contenir un tissu cornéen 6.

En variante et comme illustré à la figure 4B, l'entretoise 3 peut comprendre une pluralité d'orifices principaux 32, 33. Dans ce cas, chaque orifice principal 32, 33 est destiné à recevoir un tissu cornéen 6 respectif. Ainsi, le dispositif de maintien peut comprendre une pluralité de logements destinés à contenir chacun un tissu cornéen 6 respectif.

De préférence, l'orifice principal 31, 32, 33 (ou chaque orifice) est circulaire, le diamètre de l'orifice étant sensiblement égal au diamètre du tissu cornéen 6 pour contraindre latéralement celui-ci.

Avantageusement, l'entretoise 3 peut également comprendre un (ou plusieurs) compartiment(s) de dégazage 34. Ce (ou ces) compartiment(s) de dégazage 34 permet(tent) un stockage des bulles de gaz formées dans le tissu cornéen 6 lors de l'application d'un rayonnement électromagnétique en vue de sa découpe. En effet pour découper un fragment du tissu cornéen, un faisceau électromagnétique généré par un L.A.S.E.R. femtoseconde (délivrant des impulsions ultra-brèves et de forte puissance) peut être utilisé (au tout autre LASER connu de l'homme du métier, par exemple un L.A.S.E.R. U.V. dit *« laser à Excimer»).* A chaque impulsion, le L.A.S.E.R. femtoseconde génère un faisceau. Ce faisceau est focalisé (en un point dit « *de focalisation »* situé) dans le tissu cornéen 6. Une bulle de gaz se forme au point de focalisation, engendrant une disruption très localisée des tissus environnants. Pour former un plan de découpe dans le tissu cornéen 6, une succession de petites bulles de gaz adjacentes sont générées en déplaçant le faisceau. Ainsi, des bulles de gaz sont formées dans le tissu cornéen 6 lors de la formation d'un fragment. Le (ou les) compartiment(s) de dégazage 34 permet(tent) un stockage de ces bulles de gaz en dehors du tissu cornéen 6.

Chaque compartiment 34 peut être connecté à un (ou plusieurs) orifice(s) principal (principaux) 31, 32, 33 par l'intermédiaire d'un (ou plusieurs) canal (canaux) de connexion 35. Ce (ou ces) canal (canaux) 35 permet(tent) l'acheminement des bulles de gaz formées dans le tissu cornéen 6 vers le compartiment de dégazage 34.

Dans les modes de réalisation illustrés aux figures 4a et 4B, deux compartiments de dégazage 34 sont associés à un orifice principal 31, 32, 33 respectif. Avantageusement, ces compartiments de dégazage 34 sont diamétralement opposés pour faciliter l'évacuation de l'intégralité des bulles formées dans le tissu cornéen 6. Bien entendu, plus de deux (ou moins de deux) compartiments de dégazage (notamment trois, quatre, etc.) peuvent être associés à chaque orifice principal 31, 32, 33.

Les compartiments de dégazage 34 peuvent être sensiblement circulaires pour faciliter la fabrication (notamment le démoulage) de l'entretoise 3. Toutefois, d'autres formes peuvent être prévues pour les compartiments de dégazage (forme oblongue, rectangulaire, etc.).

### 1.3. Joint périphérique

Le joint périphérique 4 permet d'assurer l'étanchéité latérale du dispositif une fois les plaques transparentes 1, 2 assemblées, en particulier au niveau des bords 11, 21 des première et deuxième plaques 1, 2.

Avantageusement, le joint périphérique 4 est réalisé dans un matériau élastomère biocompatible et stérilisable, par exemple à base de silicone.

Dans le mode de réalisation illustré à la figure 5, le joint périphérique 4 est de forme annulaire. Toutefois, il est bien évident pour l'homme du métier que le joint périphérique 4 peut présenter d'autres formes (carrée, rectangulaire, triangulaire, etc.), notamment en fonction de la forme des première et deuxième plaques 1, 2.

L'épaisseur du joint périphérique 4 et l'épaisseur de l'entretoise 3 définissent la distance entre les première et deuxième plaques 1, 2, et donc la contrainte mécanique d'épaisseur appliquée sur le tissu cornéen 6 par lesdites plaques 1, 2. Des joints 4 et entretoises 3 de plusieurs épaisseurs peuvent être prévus pour adapter l'épaisseur du dispositif à l'épaisseur du tissu cornéen 6, ou à des choix spécifiques de l'utilisateur. Notamment dans un mode de réalisation, l'épaisseur du joint périphérique (et/ou de l'entretoise) peut être comprise entre 100 µm et 1800 µm, préférentiellement entre 400 µm et 1500 µm, et encore plus préférentiellement entre 500 µm et 700 µm. Ceci permet d'espacer les première et deuxième plaques d'une distance garantissant la contrainte mécanique des faces antérieure et postérieure du tissu cornéen 6 afin assurer le blocage en position du tissu cornéen 6 dans le dispositif de maintien.

En variante, le joint 4 et l'entretoise 3 peuvent être réalisés dans une matière expansible et/ou compressible permettant de s'adapter à différentes distances entre les première et deuxième plaques 1, 2. Dans ce cas, le réglage de cette distance est assuré par le système de blocage 51, 52 afin que les première et deuxième plaques soient espacées d'une distance comprise entre 100 µm et 1800 µm, préférentiellement entre 400 µm et 1500 µm, et encore plus préférentiellement entre 500 µm et 700 µm.

### 1.4. Système de blocage

Le système de blocage 51, 52 permet de maintenir en position l'assemblage composé des première et deuxième plaques 1, 2, du joint 4 et de l'éventuelle entretoise 3. Il garantit la stabilité de cet assemblage et permet sa manipulation par l'utilisateur sans risque d'ouverture intempestive.

Le système de blocage 51 ,52 est amovible pour permettre :
- l'insertion du tissu cornéen 6 dans le dispositif préalablement à sa découpe,
- la récupération des fragments une fois le tissu cornéen 6 découpé.

En référence à la figure 1, le système de blocage peut comprendre un cadre destiné à encercler les bords 11, 21 des première et deuxième plaques 1, 2. Le cadre peut être composé en deux parties, en particulier le cadre peut inclure des première et deuxième portions 51, 52.

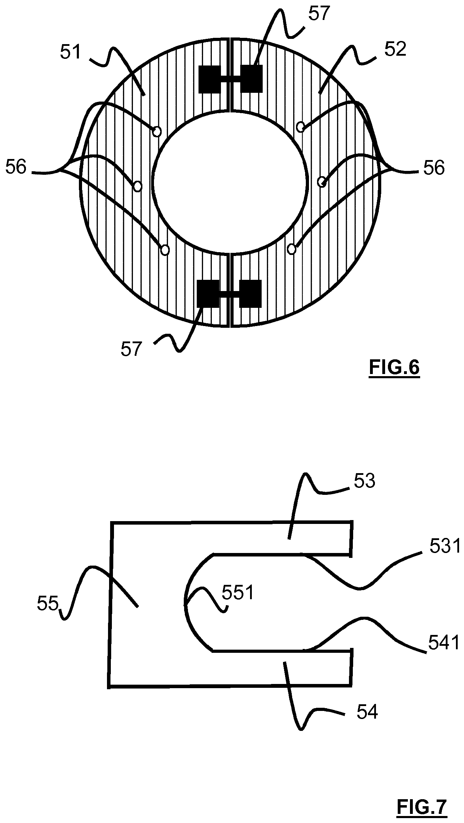

Chaque portion 51, 52 consiste par exemple en un demi-cylindre incluant une lumière traversante centrale s'étendant le long de l'axe longitudinal A-A' du dispositif. Plus précisément et en référence à la figure 7, chaque portion comprend des plateaux annulaires supérieur et inférieur 53, 54 et une paroi latérale 55 :
- la face interne 531 du plateau supérieur 53 est destinée à venir en regard de la face externe de la première plaque 1,
- la face interne 541 du plateau inférieur 54 est destinée à venir en regard de la face externe de la deuxième plaque 2, et
- la face interne 551 de la paroi latérale 55 est destinée à venir en regard des bords 11, 21 des plaques 1, 2 et de la face externe du joint 4.

Le plateau supérieur 53 et/ou le plateau inférieur 54 de chaque portion 51, 52 peut comprendre un (ou plusieurs) trou(s) 56 pour le passage d'un élément de serrage - tel qu'une vis ayant une tige filetée - permettant d'appliquer une force selon la direction longitudinale A-A' et tendant à plaquer les plaques 1, 2 contre le joint 4 et l'entretoise 3. Cet élément de fixation permet en outre de régler la distance désirée entre les première et deuxième plaques 1, 2 (et donc la contrainte mécanique appliquée sur le tissu cornéen 6).

Les première et deuxième portions 51, 52 peuvent également comprendre des moyens de fixation 57 pour permettre la solidarisation desdites portions ensemble.

En variante et comme illustré à la figure 3, le système de blocage peut comprendre des premier et deuxième cadres élémentaires :
- le premier cadre élémentaire comprend des première et deuxième portions élémentaires 51', 52' destinées à encercler le bord 11 de la première plaque 1, et
- le deuxième cadre élémentaire comprend des première et deuxième portions 51", 52" destinées à encercler le bord 21 de la deuxième plaque 2.

Dans ce cas, les premier et deuxième cadres élémentaires sont solidarisés par l'intermédiaire d'une pluralité de vis micrométriques 58 permettant de régler l'écartement entre les premier et deuxième cadre élémentaires, et donc l'écartement entre les première et deuxième plaques 1, 2, de sorte que la distance entre les première et deuxième plaques soit comprise entre 100 µm et 1800 µm, préférentiellement entre 400 µm et 1500 µm, et encore plus préférentiellement entre 500 µm et 700 µm.

### 2. Principe de fonctionnement

Le principe de fonctionnement du dispositif de maintien décrit précédemment est le suivant.

On suppose que les différents éléments constituant le dispositif de maintien ont été préalablement stérilisés.

L'utilisateur positionne la deuxième plaque 2 sur un support. Il installe l'entretoise 3 sur la deuxième plaque 2. Il positionne le joint périphérique 4 autour de l'entretoise 3.

Une fois ces trois éléments assemblés, l'utilisateur insère le tissu cornéen 6 dans l'orifice principal 31 de l'entretoise 3. Un liquide ou gel peut être introduit dans l'orifice principal 31 (autour de la cornée) pour parfaire la transmission du rayonnement (notamment pour homogénéiser les indices de réfraction par exemple).

L'utilisateur place ensuite la première plaque 1 sur l'entretoise 3 et le joint 4. La transparence des première et deuxième plaques 1, 2 à la lumière permet à l'utilisateur de vérifier visuellement le positionnement correct du tissu cornéen 6 dans l'orifice principal 31.

L'utilisateur emmanche ensuite en force les première et deuxième portions 51, 52 du système de blocage autour des bords 11, 21 des première et deuxième plaques 1, 2.

L'utilisateur actionne les moyens de fixation 57 des première et deuxième portions 51, 52 pour les solidariser entre elles.

Dans un exemple de réalisation comportant un système de blocage avec écartement réglable par vis micrométriques, l'utilisateur agit ensuite sur le (ou les) élément(s) de serrage pour régler la distance entre les première et deuxième plaques 1, 2.

Le dispositif de maintien est alors assemblé : le tissu cornéen 6 est contraint entre les première et deuxième plaques 1, 2.

Le dispositif de maintien est fixé sur un support positionné en regard d'une source de rayonnement électromagnétique, éventuellement en position verticale pour faciliter l'évacuation des bulles de gaz (susceptibles de se former lors de la découpe du tissu cornéen) vers le (ou les) compartiment de dégazage 34.

L'utilisateur actionne ensuite la source de rayonnement électromagnétique pour réaliser une pluralité de plans de découpe dans tissu cornéen 6.

Une fois les plans de découpes effectués, l'utilisateur désengage les éléments de serrage et les moyens de fixation 57. Il retire le système de blocage et ôte la première plaque 1. Il peut alors récupérer le tissu cornéen traité 6.

### 3. Conclusions

Le dispositif décrit précédemment permet l'action d'un instrument de photonique - par exemple une source L.A.S.E.R. de découpe ou de traitement - dans un tissu cornéen humain ou animal ex-vivo, en passant par les faces épithéliale (surface externe) et endothéliale (surface interne) du tissu cornéen.

Le dispositif permet la contention du tissu cornéen en contraignant celui-ci entre deux plaques transparentes au rayonnement électromagnétique. Cette contrainte permet :
- de maintenir le tissu cornéen immobile, même lorsque le tissu cornéen est soumis à un rayonnement lumineux,
- de maintenir le tissu cornéen dans une forme prédéfinie (plane, concave ou convexe) en fonction de l'application visée.

La contention du tissu cornéen par le dispositif permet d'obtenir une position contrainte connue en XYZ et stable. Cette stabilité de position permet de retourner le dispositif sans perte des repères, pour poursuivre une découpe initiée sur une face et terminée sur l'autre.

La contrainte mécanique appliquée au tissu cornéen par les deux plaques facilite en outre l'évacuation des bulles de gaz formées pendant l'interaction lumière/cornée (notamment lors d'un traitement au L.A.S.E.R. qui crée des bulles de cavitation lors de la formation du plasma). A ce titre, le (ou les) compartiments de dégazage permet(tent) un stockage des bulles de gaz en dehors du tissu cornéen.

Le dispositif permet la préparation de la cornée dans des conditions stériles et étanche (vase clos). Il permet en outre la manipulation du tissu cornéen une fois traité dans un environnement non stérile sans déconditionner la cornée et sans compromettre sa stérilité.

Le dispositif permet de découper à façon n'importe quelle forme au sein du tissu cornéen (appelée fragment) en utilisant un plan de coupe aussi bien horizontal qu'oblique, vertical ou de forme libre.

Le lecteur aura compris que de nombreuses modifications peuvent être apportées à l'invention décrite précédemment sans sortir matériellement des nouveaux enseignements et des avantages décrits ici.

Notamment dans les modes de réalisation présentés, le dispositif de maintien comprend un ou deux logements destinés à recevoir chacun un tissu oculaire. Il est bien évident pour l'homme du métier que le dispositif peut comprendre plus de deux logements (notamment trois, quatre, cinq, etc.).

Egalement, il est bien évident pour l'homme du métier que le dispositif peut être dépourvu d'entretoise.

## Revendications

1. Dispositif de maintien d'un tissu cornéen (6) humain ou animal préalablement prélevé pour son traitement photonique à partir d'un rayonnement électromagnétique, le dispositif de maintien comprenant:
- un empilement d'éléments selon un axe longitudinal (A-A') du dispositif, ledit empilement comportant :
∘ une première plaque (1) transparente au rayonnement électromagnétique,
∘ un joint périphérique (4) positionné sur la première plaque (1), le joint périphérique (4) étant destiné à s'étendre autour du tissu cornéen (6),
∘ une deuxième plaque (2) transparente au rayonnement électromagnétique sur le joint périphérique (4),
**caractérisé en ce que** le dispositif de maintien comprend :
- un système de blocage (51, 52) de l'empilement d'éléments apte à plaquer les première et deuxième plaques (1, 2) contre le tissu cornéen (6) pour appliquer une contrainte mécanique sur des faces antérieure et postérieure du tissu cornéen (6).

2. Dispositif de maintien selon la revendication 1, ***dans lequel*** les première et deuxième plaques sont parallèles, la distance entre les première et deuxième plaques étant comprise entre 100 µm et 1800 µm, préférentiellement entre 400 µm et 1500 µm, et encore plus préférentiellement entre 500 µm et 700 µm.

3. Dispositif de maintien selon l'une quelconque des revendications 1 ou 2, ***dans lequel*** l'épaisseur du joint périphérique (4) est comprise entre 100 µm et 1800 µm, préférentiellement entre 400 µm et 1500 µm, et encore plus préférentiellement entre 500 µm et 700 µm, ladite épaisseur du joint définissant la distance entre les première et deuxième plaques (1, 2) représentative de la contrainte mécanique appliquée sur le tissu cornéen (6) par lesdites plaques (1, 2).

4. Dispositif de maintien selon la revendication 3, ***dans** lequel* le système de blocage (51, 52) de l'empilement d'éléments est apte à plaquer les première et deuxième plaques (1, 2) contre le joint périphérique de sorte à espacer les première et deuxième plaques (1, 2) d'une distance comprise entre 100 µm et 1800 µm, préférentiellement entre 400 µm et 1500 µm, et encore plus préférentiellement entre 500 µm et 700 µm.

5. Dispositif de maintien selon la revendication 1 à 4, *dans lequel* le système de blocage comporte un cadre destiné à entourer au moins partiellement les bords (11, 21) des première et deuxième plaques (1, 2), le cadre étant apte à :
∘ induire sur la première plaque (1) l'application d'une force parallèle à l'axe longitudinal (A-A') et tendant à déplacer la première plaque (1) vers la deuxième plaque (2),
∘ induire sur la deuxième plaque (2) l'application d'une force parallèle à l'axe longitudinal (A-A') et tendant à déplacer la deuxième plaque (2) vers la première plaque (1).

6. Dispositif de maintien selon la revendication 5, *dans lequel* le cadre est composé d'au moins une première et deuxième portions (51, 52), chaque portion incluant un plateau supérieur (53), un plateau inférieur (54) et une paroi latérale (55) entre les plateaux supérieur et inférieur (53, 54) :
- la face interne (531) du plateau supérieur (53) étant destinée à venir en regard de la face externe de la première plaque (1),
- la face interne (541) du plateau inférieur (54) étant destinée à venir en regard de la face externe de la deuxième plaque (2), et
- la face interne (551) de la paroi latérale (55) étant destinée à venir en regard des bords (11, 21) des plaques (1, 2) et de la face externe du joint (4).

7. Dispositif de maintien selon la revendication 6, ***dans lequel*** le système de blocage comporte en outre au moins un élément de serrage, tel qu'une vis (58), chaque élément de serrage étant destiné à coopérer avec un trou (56) respectif ménagé dans le plateau supérieur (53) et/ou dans le plateau inférieur (54) d'une portion (51, 52) du cadre.

8. Dispositif de maintien selon la revendication 7, ***dans lequel*** chaque élément de serrage est déplaçable pour régler la distance entre les première et deuxième plaques, ladite distance étant comprise entre 100 µm et 1800 µm, préférentiellement entre 400 µm et 1500 µm, et encore plus préférentiellement entre 500 µm et 700 µm.

9. Dispositif de maintien selon l'une quelconque des revendications 6 à 8, ***dans lequel*** les première et deuxième portions (51, 52) de cadre incluent des moyens de fixation (57) pour permettre la solidarisation desdites portions ensemble.

10. Dispositif de maintien selon l'une quelconque des revendications 1 à 9, ***lequel*** comprend en outre une entretoise (3) positionnée entre les première et deuxième plaques (1, 2), le joint périphérique (4) s'étendant autour de l'entretoise (3).

11. Dispositif de maintien selon la revendication 10, ***dans lequel*** l'épaisseur de l'entretoise (3) est comprise entre 100 µm et 1800 µm, préférentiellement entre 400 µm et 1500 µm, et encore plus préférentiellement entre 500 µm et 700 µm, ladite épaisseur de l'entretoise (3) définissant la distance entre les première et deuxième plaques (1, 2) représentative de la contrainte mécanique appliquée sur le tissu cornéen (6) par lesdites plaques (1, 2), ladite distance étant comprise entre 100 µm et 1800 µm, préférentiellement entre 400 µm et 1500 µm, et encore plus préférentiellement entre 500 µm et 700 µm.

12. Dispositif de maintien selon la revendication précédente, ***dans lequel*** l'entretoise (3) comporte au moins un orifice principal (31, 32, 33) définissant, avec les faces internes des première et deuxième plaques (1, 2), un logement destiné à contenir le tissu cornéen (6).

13. Dispositif de maintien selon l'une quelconque des revendications 10 à 12, ***dans lequel*** l'entretoise (3) comporte au moins un compartiment de dégazage (34).

14. Dispositif de maintien selon les revendications 12 et 13 prises en combinaison, ***dans lequel*** l'entretoise (3) comporte au moins un canal de connexion (35) s'étendant entre l'orifice principal (31, 32, 33) et le compartiment de dégazage (34) pour l'acheminement de bulles de gaz formées dans le tissu cornéen (6).

## Patentansprüche

1. Vorrichtung zur Aufnahme von zuvor entnommenem Hornhautgewebe (6) eines Menschen oder eines Tieres zwecks dessen Photonenbehandlung durch elektromagnetische Strahlung, wobei die Aufnahmevorrichtung umfasst:
- einen Elementestapel gemäß einer Längsachse (A-A') der Vorrichtung, wobei der Stapel aufweist:
o eine erste, für elektromagnetische Strahlung durchlässige Platte (1),
∘ eine Umfangsdichtung (4), die auf der ersten Platte (1) positioniert ist, wobei die Umfangsdichtung (4) bestimmt ist, sich um das Hornhautgewebe (6) zu erstrecken,
∘ eine zweite, für elektromagnetische Strahlung durchlässige Platte (2) auf der Umfangsdichtung (4),
**dadurch gekennzeichnet, dass** die Haltevorrichtung umfasst:
- ein Arretiersystem (51, 52) des Elementestapels, das imstande ist, die erste und zweite Platte (1, 2) gegen das Hornhautgewebe (6) zu drücken, um eine mechanische Kraft auf die Vorder- und Rückseite des Hornhautgewebes (6) auszuüben.

2. Aufnahmevorrichtung nach Anspruch 1, wobei die erste und zweite Platte parallel sind, wobei der Abstand zwischen der ersten und zweiten Platte zwischen 100 µm und 1800 µm, vorzugsweise zwischen 400 µm und 1500 µm und noch vorzugsweiser zwischen 500 µm und 700 µm liegt.

3. Aufnahmevorrichtung nach einem der Ansprüche 1 oder 2, wobei die Dicke der Umfangsdichtung (4) zwischen 100 µm und 1800 µm, vorzugsweise zwischen 400 µm und 1500 µm und noch vorzugsweiser zwischen 500 µm und 700 µm liegt, wobei die Dicke der Dichtung den Abstand zwischen der ersten und zweiten Platte (1, 2) definiert, der für die auf das Hornhautgewebe (6) von den Platten (1, 2) ausgeübte mechanische Kraft repräsentativ ist.

4. Aufnahmevorrichtung nach Anspruch 3, wobei das Arretiersystem (51, 52) des Elementestapels imstande ist, die erste und zweite Platte (1, 2) gegen die Umfangsdichtung derart zu drücken, dass die erste und zweite Platte (1, 2) in einem Abstand beabstandet sind, der zwischen 100 µm und 1800 µm, vorzugsweise zwischen 400 µm und 1500 µm und noch vorzugsweiser zwischen 500 µm und 700 µm liegt.

5. Aufnahmevorrichtung nach Anspruch 1 bis 4, wobei das Arretiersystem einen Rahmen aufweist, der bestimmt ist, die Ränder (11, 21) der ersten und zweiten Platte (1, 2) mindestens teilweise zu umgeben, wobei der Rahmen imstande ist:
∘ auf die erste Platte (1) die Anwendung einer Kraft zu induzieren, die parallel zur Längsachse (A-A') ist und bewirkt, dass die erste Platte (1) in Richtung der zweiten Platte (2) verlagert wird,
∘ auf die zweite Platte (2) die Anwendung einer Kraft zu induzieren, die parallel zur Längsachse (A-A') ist und bewirkt, dass die zweite Platte (2) in Richtung der ersten Platte (1) verlagert wird.

6. Aufnahmevorrichtung nach Anspruch 5, wobei der Rahmen aus mindestens einem ersten und zweiten Abschnitt (51, 52) zusammengesetzt ist, wobei jeder Abschnitt eine obere Platte (53), eine untere Platte (54) und eine Seitenwand (55) zwischen der oberen und unteren Platte (53, 54) einschließt:
- wobei die innere Fläche (531) der oberen Platte (53) bestimmt ist, der äußeren Fläche der ersten Platte (1) zugewandt zu kommen,
- wobei die innere Fläche (541) der unteren Platte (54) bestimmt ist, der äußeren Fläche der zweiten Platte (2) zugewandt zu kommen, und
- wobei die innere Fläche (551) der Seitenwand (55) bestimmt ist, den Rändern (11, 21) der Platten (1, 2) und der äußeren Fläche der Dichtung (4) zugewandt zu kommen.

7. Aufnahmevorrichtung nach Anspruch 6, wobei das Arretiersystem ferner mindestens ein Spannelement wie eine Schraube (58) aufweist, wobei jedes Spannelement bestimmt ist, mit einem jeweiligen Loch (56) zusammenzuwirken, das in der oberen Platte (53) und/oder in der unteren Platte (54) eines Abschnitts (51, 52) des Rahmens eingerichtet ist.

8. Aufnahmevorrichtung nach Anspruch 7, wobei jedes Spannelement verlagerbar ist, um den Abstand zwischen der ersten und zweiten Platte einzustellen, wobei der Abstand zwischen 100 µm und 1800 µm, vorzugsweise zwischen 400 µm und 1500 µm und noch vorzugsweiser zwischen 500 µm und 700 µm liegt.

9. Aufnahmevorrichtung nach einem der Ansprüche 6 bis 8, wobei der erste und zweite Abschnitt (51, 52) des Rahmens Befestigungsmittel (57) aufweisen, um die feste Verbindung der Abschnitte miteinander zu gestatten.

10. Aufnahmevorrichtung nach einem der Ansprüche 1 bis 9, die ferner einen Abstandshalter (3) umfasst, der zwischen der ersten und zweiten Platte (1, 2) positioniert ist, wobei sich die Umfangsdichtung (4) um den Abstandshalter (3) erstreckt.

11. Aufnahmevorrichtung nach Anspruch 10, wobei die Dicke des Abstandshalters (3) zwischen 100 µm und 1800 µm, vorzugsweise zwischen 400 µm und 1500 µm und noch vorzugsweiser zwischen 500 µm und 700 µm liegt, wobei die Dicke des Abstandshalters (3) den Abstand zwischen der ersten und zweiten Platte (1, 2) definiert, der für die auf das Hornhautgewebe (6) von den Platten (1, 2) ausgeübte mechanische Kraft repräsentativ ist, wobei der Abstand zwischen 100 µm und 1800 µm, vorzugsweise zwischen 400 µm und 1500 µm und noch vorzugsweiser zwischen 500 µm und 700 µm liegt.

12. Aufnahmevorrichtung nach vorangehendem Anspruch, wobei der Abstandshalter (3) mindestens eine Hauptöffnung (31, 32, 33) aufweist, die mit den inneren Flächen der ersten und zweiten Platte (1, 2) eine Aufnahme definiert, die bestimmt ist, das Hornhautgewebe (6) zu enthalten.

13. Aufnahmevorrichtung nach einem der Ansprüche 10 bis 12, wobei der Abstandshalter (3) mindestens ein Gasableitungsfach (34) aufweist.

14. Aufnahmevorrichtung nach den Ansprüchen 12 und 13 in Kombination, wobei der Abstandshalter (3) mindestens einen Verbindungskanal (35) aufweist, der sich zwischen der Hauptöffnung (31, 32, 33) und dem Gasableitungsfach (34) zur Beförderung von im Hornhautgewebe (6) ausgebildeten Gasblasen erstreckt.

## Claims

1. A device for holding human or animal corneal tissue (6) previously removed for photonic treatment thereof from electromagnetic radiation, the holding device comprising:
- a stack of elements along a longitudinal axis (A-A') of the device, said stack comprising:
∘ a first plate (1) transparent to electromagnetic radiation,
∘ a peripheral seal (4) positioned on the first plate (1), the peripheral seal (4) being intended to extend around the corneal tissue (6),
∘ a second plate (2) transparent to electromagnetic radiation on the peripheral seal (4),
**characterised in that** the holding device comprises:
- a locking system (51, 52) of the stack of elements capable of pressing the first and second plates (1, 2) against the corneal tissue (6) to apply mechanical stress to the anterior and posterior faces of the corneal tissue (6).

2. The holding device according to claim 1, ***wherein*** the first and second plates are parallel, the distance between the first and second plates being comprised between 100 µm and 1800 µm, preferably between 400 µm and 1500 µm, and more preferably between 500 µm and 700 µm.

3. The holding device according to any one of claims 1 or 2, ***wherein*** the thickness of the peripheral seal (4) is comprised between 100 µm and 1800 µm, preferably between 400 µm and 1500 µm, and more preferably between 500 µm and 700 µm, said thickness of the seal defining the distance between the first and second plates (1, 2) representative of the mechanical stress applied to the corneal tissue (6) by said plates (1, 2).

4. The holding device according to claim 3, ***wherein*** the locking system (51, 52) of the stack of elements is capable of pressing the first and second plates (1, 2) against the peripheral seal so as to space the first and second plates (1, 2) at a distance comprised between 100 µm and 1800 µm, preferably between 400 µm and 1500 µm, and more preferably between 500 µm and 700 µm.

5. The holding device according to claims 1 to 4, ***wherein*** the locking system comprises a frame for at least partially surrounding the edges (11, 21) of the first and second plates (1, 2), the frame being able to:
∘ induce on the first plate (1) the application of a force parallel to the longitudinal axis (A-A') and tending to move the first plate (1) toward the second plate (2),
∘ induce on the second plate (2) the application of a force parallel to the longitudinal axis (A-A') and tending to move the second plate (2) toward the first plate (1).

6. The holding device according to claim 5, ***wherein*** the frame is composed of at least a first and a second portions (51, 52), each portion including an upper tray (53), a lower tray (54) and a side wall (55) between the upper and lower trays (53, 54):
- the inner face (531) of the upper tray (53) being intended to come facing the outer face of the first plate (1),
- the inner face (541) of the lower tray (54) being intended to come facing the outer face of the second plate (2), and
- the inner face (551) of the side wall (55) being intended to come facing the edges (11, 21) of the plates (1, 2) and the outer face of the seal (4).

7. The holding device according to claim 6, ***wherein*** the locking system further comprises at least one clamping element, such as a screw (58), each clamping element being intended to cooperate with a respective hole (56) provided in the upper tray (53) and/or in the lower tray (54) of a portion (51, 52) of the frame.

8. The holding device according to claim 7, ***wherein*** each clamping element is movable to adjust the distance between the first and second plates, said distance being comprised between 100 µm and 1800 µm, preferably between 400 µm and 1500 µm, and more preferably between 500 µm and 700 µm.

9. The holding device according to any one of claims 6 to 8, ***wherein*** the first and second portions (51, 52) of the frame include fastening means (57) to enable said portions to be joined together.

10. The holding device according to any one of claims 1 to 9, ***which*** further comprises a spacer (3) positioned between the first and second plates (1, 2), the peripheral seal (4) extending around the spacer (3).

11. The holding device according to claim 10, *wherein* the thickness of the spacer (3) is comprised between 100 µm and 1800 µm, preferably between 400 µm and 1500 µm, and more preferably between 500 µm and 700 µm, said thickness of the spacer (3) defining the distance between the first and second plates (1, 2) representative of the mechanical stress applied to the corneal tissue (6) by said plates (1, 2), said distance being comprised between 100 µm and 1800 µm, preferably between 400 µm and 1500 µm, and more preferably between 500 µm and 700 µm.

12. The holding device according to the preceding claim, ***wherein*** the spacer (3) comprises at least one main opening (31, 32, 33) defining, with the inner faces of the first and second plates (1, 2), a housing intended to contain the corneal tissue (6).

13. The holding device according to any one of claims 10 to 12, *wherein* the spacer (3) comprises at least one degassing compartment (34).

14. The holding device according to claims 12 and 13 in combination, *wherein* the spacer (3) comprises at least one connecting channel (35) extending between the main opening (31, 32, 33) and the degassing compartment (34) for the delivery of gas bubbles formed in the corneal tissue (6).
